(19) Europäisches Patentamt
European Patent Office
Office européen des brevets

(11) **EP 4 243 031 A1**

(12) **EUROPEAN PATENT APPLICATION**

(43) Date of publication:
**13.09.2023 Bulletin 2023/37**

(21) Application number: **23161235.9**

(22) Date of filing: **10.03.2023**

(51) International Patent Classification (IPC):
**G16H 20/17** (2018.01)  **A61B 5/145** (2006.01)
**A61B 5/00** (2006.01)  **A61M 5/172** (2006.01)
**G16H 40/67** (2018.01)  **G16H 50/20** (2018.01)
**G16H 40/63** (2018.01)

(52) Cooperative Patent Classification (CPC):
**G16H 20/17; A61B 5/14532; A61B 5/4839;
A61M 5/1723; G16H 40/63; G16H 40/67;
G16H 50/20;** A61M 2202/0486

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.03.2022 FR 2202161**

(71) Applicant: **Diabeloop
38000 Grenoble (FR)**

(72) Inventors:
• **DESIR, Chesner
  38000 GRENOBLE (FR)**
• **LACHAL, Sylvain
  38600 JEAN PAIN (FR)**
• **TOURKI, Yousra
  92800 PUTEAUX (FR)**

(74) Representative: **Fidal Innovation
4-6 avenue d'Alsace
92400 Courbevoie (FR)**

(54) **COMPUTERIZED SYSTEM FOR COMMUNICATING TREATMENT INFORMATION TO A USER**

(57) A computerized system for communicating treatment information to a user. The computerized system comprises a memory (22) storing data comprising least time-based user metabolite data and a processor (23). The processor (23) comprising a dose determination module (24) adapted to determine a possible drug dosage based on said data, a disturbance module (25) adapted to determine whether said data or the possible drug dosage triggers at least a disturbance threshold from a plurality of disturbance thresholds and a notification module (26) adapted to, when the disturbance module (25) determines that the at least one disturbance threshold is triggered or when at least a disturbance bypass is triggered, issue a user notification, the user notification being issued to the user and comprising the possible drug dosage. The plurality of disturbance thresholds comprising a bolus threshold, a drug threshold, and a time threshold.

[Fig. 1]

**EP 4 243 031 A1**

**Description**

**FIEOD OF THE INVENTION**

The present invention relates to a computerized system for communicating treatment information to a user.

**TECHNOLOGICAL BACKGROUND**

More specifically, the present invention relates to a computerized system for communicating treatment information to a user and associated method.

As stated in the document US7713229B2, medication infusion devices and physiological fluid characteristic monitoring devices are known in the medical field. One very common application of such devices is the delivery of insulin to and the monitoring of blood glucose levels of diabetic patients. Increased portability and ease of use of such devices have enabled the diabetic patient to administer a self-regulated medical treatment regime which in turn provides an increased level of patient autonomy and privacy. This is particularly beneficial since a diabetic patient's glucose levels may vary frequently during the day.

Thus, it is important to provide the patient with information in a smart manner. The document US9439602B2 describes a glucose monitoring system alerting a user based on clinical risk,

as a measure of the severity of a specific glycemic condition, which depends mainly on the glucose level, but might be influenced also by other factors, such as glucose trend and possibly the quantity of insulin present in the patient's body. In particular, when evaluating the "clinical risk" to which the patient is exposed, one should consider not only the glycemic level but also its trend. Notably, a device which raises alarms on the basis of glucose sensor information and critical risk, should exploit technologies embeddable on a small PDA platform.

However, the document US9439602B2 does not take into consideration the mental burden implied by a system only taking into account an arbitrary clinical risk.

The present invention aims at providing a computerized system for communicating treatment information to a user and associated method able to issue notification to the user while reducing said mental burden.

**SUMMARY OF THE INVENTION**

The present invention relates to a computerized system for communicating treatment information to a user, wherein the computerized system comprises:

. a memory storing data comprising, at least, time-based user metabolite data;
. a processor comprising:

- a dose determination module adapted to determine a possible drug dosage based on said data;
- a disturbance module adapted to determine whether said data or the possible drug dosage triggers at least a disturbance threshold from a plurality of disturbance thresholds;
- a notification module adapted to, when the disturbance module determines that the at least one disturbance threshold is triggered or when at least a disturbance bypass is triggered, issue a user notification, the user notification being issued to the user and comprising the possible drug dosage;

wherein the plurality of disturbance thresholds comprises:

. a bolus threshold hereinafter referred as BT, the BT corresponding to a minimal possible drug dosage;
. a drug threshold hereinafter referred as IT, the IT corresponding to a minimal possible drug dosage; and
. a time threshold hereinafter referred as TT, the TT corresponding to a minimal amount of time elapsed since a previous user notification issued to a user or a previous bolus administered to the user.

According to the present invention, a disturbance bypass is a situation in which the user must receive treatment information as soon as possible and therefore cannot wait for at least one disturbance threshold to be triggered. A disturbance bypass could be a situation where the user is facing a severe hyperglycemia for example.

According to the present invention, the drug dosage corresponds to an insulin dosage recommendation.

According to the present invention, to issue a user notification means sending a notification to a user. Said user notification can be accompanied by notifying elements such as any type of sound, light, or vibration. According to an embodiment, the more important the user notification, the more it will be accompanied by notifying elements.

According to an embodiment, the time-based user metabolite data are provided by a Continuous Glucose Monitoring sensor.

According to an embodiment, the possible drug dosage determined by the dose determination module is viewable at any time by the user.

According to the present invention, the drug threshold is an insulin threshold.

Having at least one disturbance bypass allows to issue a user notification to the user in different situations and therefore allows to improve the security of the user.

Having a BT allows to issue a user notification and therefore disturb the user only if the determined possible drug dosage is higher than a minimum administrable amount of insulin using the favorite administration method and device of the user. Indeed, some devices only allow administering a single unit of insulin, therefore, it is not relevant to issue a user notification and to disturb the user for a determined possible drug dosage that cannot be administered to said user.

According to an embodiment wherein the system is connected to a dispensing device such as a smart insulin pen, the BT is a predetermined value corresponding to the minimum administrable amount of insulin using the administration method and dispensing device.

Having an IT allows to issue a user notification and therefore disturb the user only to noticeably correct the glycemia. For example wherein the user is insulin deprived, an IT could be equal to an amount of insulin having the effect of lowering the user glycemia by three mg/dl. Therefore, if a possible drug dosage is calculated to correct the glycemia of a value inferior to three mg/dl, the IT would not be triggered thus no user notification would be issued and the user would not be disturbed.

According to an embodiment, the IT is a predetermined value corresponding to a user choice.

Having a TT allows to issue a user notification and therefore disturb the user only after a certain amount of time has elapsed since the last issued user notification and/or since the last bolus received by the user and therefore reducing the global disturbance of the system.

According to the present invention, a bolus received corresponds to a certain dose of insulin injected/administered in the user body.

According to a preferred embodiment, the TT corresponds to a minimal amount of time elapsed since previous bolus has been administered to the user.

According to an embodiment, the notification module is adapted to, when the disturbance module determines that at least the BT and the TT are triggered or when at least one disturbance bypass is triggered, issue a user notification.

Such a configuration allows to potentially minimize the global disturbance of the system as it potentially reduces the number of user notifications issued according to both the BT and the IT.

According to an embodiment, the notification module is adapted to, when the disturbance module determines that at least the BT, the IT and the TT are triggered or when at least one disturbance bypass is triggered, issue a user notification.

Such a configuration allows to potentially minimize the global disturbance of the system as it potentially reduces the number of user notifications issued according to the BT, the IT and the TT.

According to an embodiment, the IT depends on a minimum glycemia to correct and a compensation factor.

According to an embodiment, IT is as follows:

$$[\text{Math 1}] \quad IT = \Delta G_{min} \times \frac{CF}{100}$$

; wherein

$\Delta G_{min}$ represents a minimum glycemia to correct; and
CF represents a compensation factor [U/g/L].

Such a configuration allows to accurately determine the IT.

According to an embodiment, the compensation factor is derived from the user Total Daily Dose of insulin hereinafter referred as TDD with

$$[\text{Math 2}] \quad CF = \frac{TDD}{18}.$$

Such a configuration allows to accurately determine the compensation factor.

According to an embodiment, the BT decreases depending on the amount of time elapsed since a previous user notification has been issued to a user or a previous bolus has been administered to the user.

According to a preferred embodiment, the BT decreases depending on the amount of time elapsed since a previous bolus has been administered to the user.

Such a configuration allows to maintain a good balance between user security and his disturbance. Indeed, having a BT decreasing depending on the amount of time elapsed since a previous bolus has been administered to the user allows to issue a user notification to the user even if a short amount of time has elapsed since the previous user notification issued or the previous bolus administered if the possible drug dosage is way greater than the IT or if a user glycemia might be outside an acceptable range in the future for example.

According to an embodiment, the BT decreases from a maximum insulin value, said maximum insulin value depending on the physiological impact of a unit of insulin to the user. Such a configuration allows the system to adapt to the user. According to an embodiment, the BT decreases from a maximum value, said maximum insulin value being equal to

$$[\text{Math 3}] \quad min(xU, xCF)$$

. Where xU corresponds to a maximum amount of insulin units and xCF being a compensation factor multiplied by a constant in order to take into account the insulin needs of the user.

According to an embodiment, xU is a pre-registered value comprised between two and eight units of insulin.

According to an embodiment, the BT has a minimum insulin value after a certain amount of time elapsed since a previous user notification has been issued to a user or a previous bolus has been administered to the user.

According to a preferred embodiment, the BT cannot decrease further than a minimum insulin value. BT is equal to said minimum insulin value after a certain amount of time elapsed since a previous bolus has been administered to the user.

Such a configuration allows to maintain a good balance between user security and his disturbance. Indeed, having a minimal value after a certain amount of time allows to not issue a user notification comprising a drug dosage too low to be relevant, even if the time elapsed since the last user notification issued or bolus administered is way greater than the TT.

According to an embodiment, the BT has a minimum insulin value, said minimum insulin value depending on the physiological impact of a unit of insulin to the user and a user preference. Such a configuration allows the system to adapt to the user.

According to an embodiment, the BT has a minimum insulin value, said minimum insulin value being equal to

$$[\text{Math 4}]\ max(yU, pU, yCF)$$

. Where yU corresponds to a minimum amount of insulin units that could, in a specific embodiment, be related to the favorite administration method and device of the user, pU corresponds to an amount of insulin units according to a user preference and yCF being a compensation factor multiplied by a constant in order to take into account the insulin needs of the user.

According to an embodiment, yU is a pre-registered value comprised between half a unit of insulin and 2 units of insulin.

According to an embodiment, BT depends on a maximum insulin value, a maximal glycemia to correct, a minimal insulin value, an insulin value taking into account the compensation factor and a secondary compensation factor that may be equal to the compensation factor. A secondary compensation factor that may be equal to the compensation factor allows the system to further adapt to the user.

Such a preferred embodiment allows to maintain a better balance between user security and his disturbance.

According to an embodiment, a disturbance bypass is an emergency bypass, said emergency bypass being triggered wherein a risk for the user is identified from the data.

Such a configuration allows to bypass the trigger of any threshold and therefore the security of the user.

According to an embodiment, an emergency might be when a determined and/or estimated current insulin on board of the user is under a certain value. Therefore, the notification module is adapted to issue a user notification comprising a possible drug dosage when the current insulin on board of the user is under a certain value. According to an embodiment, the insulin on board is determined and/or estimated based on the insulin previously injected in the user, a consumption of insulin over time and the time.

According to an embodiment, the insulin on board is determined using, at least, a mathematical model representing the evolution of insulin on board over time based on the insulin previously injected in the user, a consumption of insulin over time and the time.

The present invention also relates to a method for communicating treatment information to a user, wherein the method is implemented by a computerized system and comprises the following steps:

- data reception, the step of data reception consisting of receiving data comprising at least time-based user metabolite data;
- drug dosage determination , the step of drug dosage determination consisting of determining a possible drug dosage based on said data;
- trigger determination, the step of trigger determination consisting of determining whether said data or the possible drug dosage triggers at least a disturbance threshold from a plurality of disturbance thresholds; and
- issuing notification, the step of issuing notification consisting of issuing a user notification to a user when it is determined, during the step of trigger determination , that at least one threshold from the plurality of disturbance thresholds is triggered or when at least a disturbance bypass is triggered, the user notification comprising the possible drug dosage;

wherein the plurality of disturbance thresholds comprises:

. a bolus threshold hereinafter referred as BT, the BT corresponding to a minimal bolus dose;
. a drug threshold hereinafter referred as IT, the IT corresponding to a minimal dose of insulin; and
. a time threshold hereinafter referred as TT, the TT corresponding to a minimal amount of time elapsed since a previous user notification issued to a user or a previous bolus administered to the user.

According to an embodiment, all the previously described characteristics and advantages applicable to the computerized system are also applicable to the method.

According to an embodiment, the method is implemented by the system for communicating treatment information to a user previously described.

According to an embodiment, the step of issuing notification consists of issuing a user notification to a user when it is determined, during the step of trigger determination, that at least the BT and the TT are triggered or when at least one disturbance bypass is triggered.

According to an embodiment, the step of issuing notification consists of issuing a user notification to a user when it is determined, during the step of trigger determination, that at least the BT, the IT and the TT are triggered or when at least one disturbance bypass is triggered.

According to an embodiment, the IT depends on a minimum glycemia to correct and a compensation factor.

According to an embodiment, the BT decreases depending on the amount of time elapsed since a previous user notification has been issued to a user or a previous bolus has been administered to the user.

According to an embodiment, the BT has a minimum value after a certain amount of time elapsed since a previous user notification has been issued to a user or a previous bolus has been administered to the user.

According to an embodiment, a disturbance bypass is an emergency bypass, said emergency bypass being triggered wherein a risk for the user is identified from the data.

The present invention also relates to a computer program product comprising instructions which, when the program is executed by a computer, cause the computer to carry out the steps of the method described above.

According to the present invention the term "insulin" could be understood as being any type of drug.

**BRIEF DESCRIPTION OF DRAWINGS**

Embodiments of the invention will be described below with reference to the drawings, described briefly below:

> [Fig. 1] represents a schematic view of a computerized system for communicating treatment information to a user according to the present invention;
> [Fig. 2] represents steps of a method for communicating treatment information to a user according to the present invention; and
> [Fig. 3] represents a drug dosage system according to the present invention.

**DETAILED DESCRIPTION**

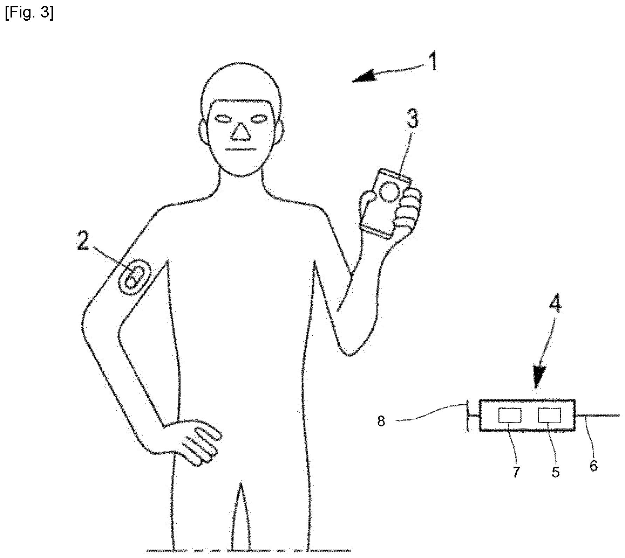

Fig. 3 shows an example of a drug dosage system 1. The drug dosage system 1 will be described in relation to an application for diabetes. However, other applications of the invention are possible. The drug dosage system 1 comprises a sensing unit 2 adapted to measure a parameter of the user such as time-based user metabolite data, in the present example, the sensing unit 2 is a Continuous Glucose Monitoring sensor. In particular, the sensing unit 2 might comprise a wearable, which is worn by the user. In addition, the sensing unit 2 is adapted to repeatedly provide a measurement of the parameter of the user. The sensing unit 2 may comprise a clock, so that time data is associated with measurement data. Such sensing units may be called continuous sensing units. The sensing unit 2 may be adapted to perform in vivo measurements. Hence, the measured parameter may be a concentration of a metabolite, such as, for example, glucose.

The sensing unit 2 comprises a data communication device adapted to communicate data to a remote system. The data communication device might be adapted to communicate wirelessly, for example using a short-range radio communication corresponding to the Bluetooth® standard or BLE standard at the priority date of the patent application.

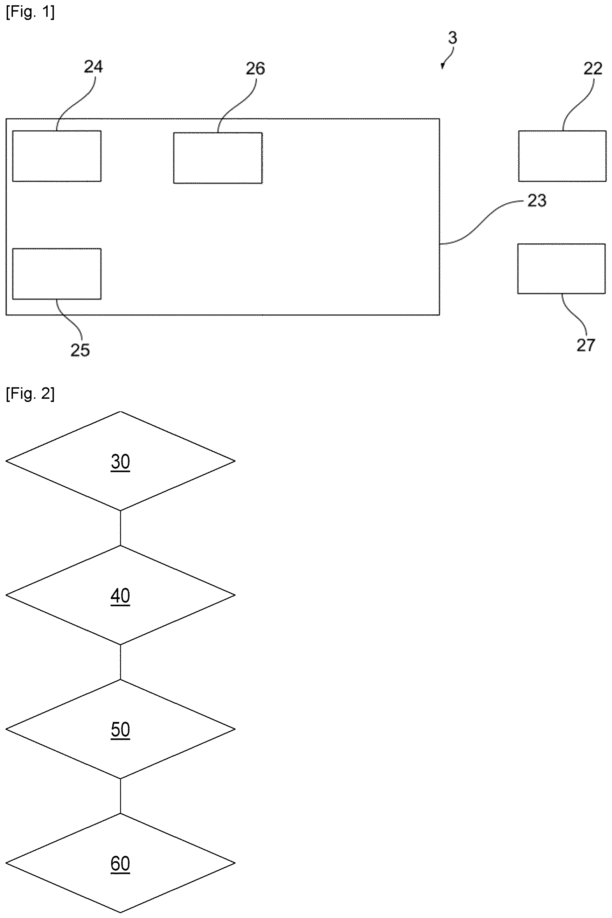

The drug dosage system 1 further comprises a terminal 3. The terminal 3 comprises a computerized system 1 for communicating treatment information to a user. Said computerized system 1, as shown on Fig. 1, comprises a processor 23 and a memory 22. The terminal 3 comprises a data communication module 27 adapted to communicate data to a remote system. The data communication module 27 might be adapted to communicate wirelessly, for example using a short-range radio communication corresponding to the Bluetooth® standard at the priority date of the patent application. For example, the data communication module 27 of the terminal 3 and the data communication device of the sensing unit 2 are compatible with one another, to form a communication system, by which the terminal 3 and the sensing unit 2 communicate with one another. In particular, measurement data, and possibly time-based user metabolite data, are communicated from the sensing unit 2 to the terminal 3, and stored in the memory 22.

As described on Fig. 3, the drug dosage system 1 further comprises a drug dispensing device 4. The drug dispensing device 4 comprises a container 5 configured to contain drug. Typically, the container 5 comprises more than one dose of drug, so as to be used a plurality of times. The drug dispensing device 4 further comprises a dispensing unit 6 adapted to dispense drug to the user. This comprises for example a needle to be inserted into the body of the user, and a plunger 8 which can be pushed to expel drug from the container 5 through the needle into the user's body. The drug dispensing device 4 further comprises a setting unit 7. The setting unit 7 can be set by the user in order to control the amount of drug which may be injected at a time using the drug dispensing device 4. For example, the setting unit 7 may be set by the user at an amount of, for example, two units, and the dispensing system is actuated to dispense drug and is prevented from operating when two units have been injected. For example, a settable mechanical stop for the plunger 8 may be used. Other embodiments are possible.

However, if the setting unit 7 does not allow to set an amount of drug inferior to one unit, the user cannot be injected with less than one unit.

Fig. 1 shows an example of the computerized communicating treatment information configured to communicate treatment information such as a possible drug dosage to a user. More specifically, the drug dosage corresponds to an insulin dosage recommendation. The computerized system 1 comprises a memory 22 configured to store data sent by the sensing unit 2 comprising, at least, time-based user metabolite data and a processor 23. The processor 23 comprises a dose determination module 24 adapted to determine a possible drug dosage based on said data, a disturbance module 25 adapted to determine whether said data or the possible drug dosage triggers at least a disturbance threshold from a plurality of disturbance thresholds and a notification module 26 adapted to, when the disturbance module 25 determines that the at least one disturbance threshold is triggered or when at least a disturbance bypass is triggered, issue a user notification, the user notification being issued to the user and comprising the possible drug dosage. According to the present invention, a disturbance bypass is a situation in which the user must receive treatment information as soon as possible and therefore cannot wait for at least one disturbance threshold to be triggered. Having at least one disturbance bypass allows to issue a user notification to the user in different situations and therefore allows to improve the security of the user.

The term "to issue a user notification" specifically means "sending a notification to a user". Said user notification can be accompanied by notifying elements such as any type of sound, light, or vibration. According to an embodiment, the more important the user notification, the more it will be accompanied by notifying elements. However, when no disturbance threshold nor disturbance bypass, the possible drug dosage determined by the dose determination module 24 is viewable at any time by the user on the terminal 3.

A disturbance bypass could be a situation where the user is facing a severe hyperglycemia for example. A disturbance bypass is an emergency bypass, said emergency bypass being triggered wherein a risk for the user is identified from the data. Such a configuration allows to bypass the trigger of any threshold and therefore the security of the user. According to an embodiment, an emergency might be when a determined and/or estimated current insulin on board of the user is under a certain value. Therefore, the notification module 26 is adapted to issue a user notification comprising a possible drug dosage when the current insulin on board of the user is under a certain value. According to an embodiment, the insulin on board is determined and/or estimated based on the insulin previously injected in the user, a consumption of insulin over time and the time. According to an embodiment, the insulin on board is determined using, at least, a mathematical model representing the evolution of insulin on board over time based on the insulin previously injected in the user, a consumption of insulin over time and the time.

The plurality of disturbance thresholds comprises a bolus threshold hereinafter referred as BT, the BT corresponding to a minimal bolus dose. Having a BT allows to issue a user notification and therefore disturb the user only if the determined possible drug dosage is higher than a minimum administrable amount of insulin using the favorite administration method and device of the user. Indeed, as previously stated, some devices only allow administering a single unit of insulin, therefore, it is not relevant to issue a user notification and to disturb the user for a determined possible drug dosage that cannot be administered to said user. According to an embodiment wherein the system is connected to an dispensing device 4 such as a smart insulin pen, the BT is a predetermined value corresponding to the minimum administrable amount of insulin using the administration method and dispensing device 4.

According to a preferred embodiment, the BT decreases depending on the amount of time elapsed since a previous bolus has been administered to the user. Such a configuration allows to maintain a good balance between user security and his disturbance. Indeed, having a BT decreasing depending on the amount of time elapsed since a previous bolus has been administered to the user allows to issue a user notification to the user even if a short amount of time has elapsed since the previous user notification issued or the previous bolus administered if the possible drug dosage is way greater than the IT or if a user glycemia might be outside an acceptable range in the future for example. The BT decreases from a maximum insulin value, said maximum insulin value depending on the physiological impact of a unit of insulin to the user, and is equal to

$$[Math\ 5]\quad min(xU, xCF)$$

. Where xU corresponds to a maximum amount of insulin units, xU being a pre-registered value comprised between two and eight units of insulin, and xCF being a compensation factor multiplied by a constant in order to take into account the insulin needs of the user. Such a configuration allows the system to precisely adapt to the user.

The plurality of disturbance thresholds also comprises a drug threshold, and more specifically an insulin threshold hereinafter referred as IT, the IT corresponding to a minimal dose of insulin. Having an IT allows to issue a user notification and therefore disturb the user only for a substantive amount of insulin. A substantive amount of insulin being an amount of insulin having the effect of substantially lowering the glycemia. For example wherein the user is insulin deprived, an IT could be equal to an amount of insulin having the effect of lowering the user glycemia by three mg/dl. Therefore, if a possible drug dosage is calculated to correct the glycemia of a value inferior to three mg/dl, the IT would not be triggered thus no user notification would be issued and the user would not be disturbed. According to an embodiment, the IT is a predetermined value corresponding to a user choice and/or preference.

According to a preferred embodiment, IT depends on a minimum glycemia to correct and a compensation factor. Therefore, IT is as follows:

$$[\text{Math 6}] \quad IT = \Delta G_{min} \times \frac{CF}{100};$$

wherein

$\Delta G_{min}$ represents a minimum glycemia to correct; and

CF represents a compensation factor [U/g/L].

Such a configuration allows to accurately determine the IT. According to an embodiment, the compensation factor is derived from the user Total Daily Dose of insulin hereinafter referred as TDD with

$$[\text{Math 7}] \quad CF = \frac{TDD}{18}$$

. Such a configuration allows to accurately determine the compensation factor.

The BT has a minimum insulin value after a certain amount of time elapsed since a previous bolus has been administered to the user. However, the BT cannot decrease further than a minimum insulin value. Such a configuration allows to maintain a good balance between user security and his disturbance. Indeed, having a minimal value after a certain amount of time allows to not issue a user notification comprising a drug dosage too low to be relevant, even if the time elapsed since the last user notification issued or bolus administered is way greater than the TT.

According to an embodiment, the BT has a minimum insulin value, said minimum insulin value depending on the physiological impact of a unit of insulin to the user and a user preference. Such a configuration allows the system to adapt to the user.

According to a preferred embodiment, the BT has a minimum insulin value, said minimum insulin value being equal to

$$[\text{Math 8}] \quad max(yU, pU, yCF)$$

. Where yU corresponds to a minimum amount of insulin units that could be dispensed using the dispensing device 4 and is a pre-registered value comprised between half a unit of insulin and 2 units of insulin, pU corresponds to an amount of insulin units according to a user preference and yCF being a compensation factor multiplied by a constant in order to take into account the insulin needs of the user. Such a configuration allows the system to adapt to the user. while maintaining a better balance between user security and his disturbance.

The plurality of disturbance thresholds comprises a time threshold hereinafter referred as TT, the TT corresponding to a minimal amount of time elapsed since a previous user notification issued to a user or a previous bolus administered to the user. Having a TT allows to issue a user notification and therefore disturb the user only after a certain amount of time has elapsed since the last bolus received by the user and therefore reducing the global disturbance of the system. According to the present invention, a bolus received corresponds to a certain dose of insulin injected/administered in the user body.

According to an embodiment, the notification module 26 is adapted to, when the disturbance module 25 determines that at least the BT and the TT are triggered or when at least one disturbance bypass is triggered, issue a user notification. Such a configuration allows to potentially minimize the global disturbance of the system as it potentially reduces the number of user notifications issued according to both the BT and the IT.

According to the preferred embodiment, the notification module 26 is adapted to, when the disturbance module 25 determines that at least the BT, the IT and the TT are triggered or when at least one disturbance bypass is triggered, issue a user notification. Such a configuration allows to potentially minimize the global disturbance of the system as it potentially reduces the number of user notifications issued according to the BT, the IT and the TT.

The present invention also relates to a method for communicating treatment information to a user, according to figure 3, wherein the method is implemented by the system for communicating treatment information to a user previously described and comprises the following steps:

- data reception 30, the step of data reception 30 consisting of receiving data comprising at least time-based user metabolite data;
- drug dosage determination 40, the step of drug dosage determination 40 consisting of determining a possible drug dosage based on said data;
- trigger determination 50, the step of trigger determination 50 consisting of determining whether said data or the possible drug dosage triggers at least a disturbance threshold from a plurality of disturbance thresholds; and
- issuing notification 60, the step of issuing notification 60 consisting of issuing a user notification to a user when it is determined, during the step of trigger determination 50, that at least one threshold from the plurality of disturbance thresholds is triggered or when at least a disturbance bypass is triggered, the user notification comprising the possible drug dosage;

wherein the plurality of disturbance thresholds comprises:

. a bolus threshold hereinafter referred as BT, the BT corresponding to a minimal bolus dose;
. a drug threshold hereinafter referred as IT, the IT corresponding to a minimal dose of insulin; and
. a time threshold hereinafter referred as TT, the TT corresponding to a minimal amount of time elapsed since a previous user notification issued to a user or a previous bolus administered to the user.

According to an embodiment, all the previously described characteristics and advantages applicable to the computerized system, such as specific disturbance thresholds characteristics for example, are also applicable to the method.

According to an embodiment, the step of issuing notification 60 consists of issuing a user notification to a user when it is determined, during the step of trigger determination 50, that at least the BT and the TT are triggered or when at least one disturbance bypass is triggered.

According to a preferred embodiment, the step of issuing notification 60 consists of issuing a user notification to a user when it is determined, during the step of trigger determination 50, that at least the BT, the IT and the TT are triggered or when at least one disturbance bypass is triggered.

The present invention also relates to a computer program product comprising instructions which, when the program is executed by a computer, cause the computer to carry out the steps of the method for communicating treatment information to a user as previously described.

According to an embodiment, all the previously described characteristics and advantages applicable to the computer program product, such as specific disturbance thresholds characteristics for example, are also applicable to the method.

While exemplary embodiment of the invention has been described with reference to two main embodiments, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiment disclosed as the best mode contemplated for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

**Claims**

1. Computerized system (1) for communicating treatment information to a user, wherein the computerized system (1) comprises:

   . a memory (22) storing data comprising, at least, time-based user metabolite data;
   . a processor (23) comprising:

   - a dose determination module (24) adapted to determine a possible drug dosage based on said data;
   - a disturbance module (25) adapted to determine whether said data or the possible drug dosage triggers at least a disturbance threshold from a plurality of disturbance thresholds;
   - a notification module (26) adapted to, when the disturbance module (25) determines that the at least one disturbance threshold is triggered or when at least a disturbance bypass is triggered, issue a user notification, the user notification being issued to the user and comprising the possible drug dosage;

   wherein the plurality of disturbance thresholds comprises:

   . a bolus threshold hereinafter referred as BT, the BT corresponding to a minimal possible drug dosage;
   . a drug threshold hereinafter referred as IT, the IT corresponding to a minimal possible drug dosage; and
   . a time threshold hereinafter referred as TT, the TT corresponding to a minimal amount of time elapsed since a previous user notification issued to a user or a previous bolus administered to the user.

2. Computerized system (1) for communicating treatment information to a user according to claim 1, wherein the notification module (26) is adapted to, when the disturbance module (25) determines that at least the BT and the TT are triggered or when at least one disturbance bypass is triggered, issue a user notification.

3. Computerized system (1) for communicating treatment information to a user according to claim 2, wherein the notification module (26) is adapted to, when the disturbance module (25) determines that at least the BT, the IT and the TT are triggered or when at least one disturbance bypass is triggered, issue a user notification.

4. Computerized system (1) for communicating treatment information to a user according to any of the claims 1 to 3, wherein the IT depends on a minimum glycemia to correct and a compensation factor.

5. Computerized system (1) for communicating treatment information to a user according to any of the claims 1 to 4, wherein the BT decreases depending on the amount of time elapsed since a previous user notification has been issued to a user or a previous bolus has been administered to the user.

6. Computerized system (1) for communicating treatment information to a user according to claim 5, wherein the BT has a minimum insulin value after a certain amount of time elapsed since a previous user notification has been issued to a user or a previous bolus has been administered to the user.

7. Computerized system (1) for communicating treatment information to a user according to any of the claims 1 to 6, wherein a disturbance bypass is an emergency bypass, said emergency bypass being triggered wherein a risk for the user is identified from the data.

8. A method for communicating treatment information to a user, wherein the method is implemented by a computerized system and comprises the following steps:

   - data reception (30), the step of data reception (30) consisting of receiving data comprising at least time-based user metabolite data;
   - drug dosage determination (40), the step of drug dosage determination (40) consisting of determining a possible drug dosage based on said data;
   - trigger determination (50), the step of trigger determination (50) consisting of determining whether said data or the possible drug dosage triggers at least a disturbance threshold from a plurality of disturbance thresholds; and
   - issuing notification (60), the step of issuing notification (60) consisting of issuing a user notification to a user when it is determined, during the step of trigger determination (50), that at least one threshold from the plurality of disturbance thresholds is triggered or when at least a disturbance bypass is triggered, the user notification comprising the possible drug dosage;
   wherein the plurality of disturbance thresholds comprises:

      . a bolus threshold hereinafter referred as BT, the BT corresponding to a minimal bolus dose;
      . a drug threshold hereinafter referred as IT, the IT corresponding to a minimal dose of insulin; and
. a time threshold hereinafter referred as TT, the TT corresponding to a minimal amount of time elapsed since a previous user notification issued to a user or a previous bolus administered to the user.

9. Method for communicating treatment information to a user according to claim 8, wherein the step of issuing notification (60) consists of issuing a user notification to a user when it is determined, during the step of trigger determination (50), that at least the BT and the TT are triggered or when at least one disturbance bypass is triggered.

10. Method for communicating treatment information to a user according to claim 9, wherein the step of issuing notification (60) consists of issuing a user notification to a user when it is determined, during the step of trigger determination (50), that at least the BT, the IT and the TT are triggered or when at least one disturbance bypass is triggered.

11. Method for communicating treatment information to a user according to any of the claims 8 to 10, wherein the IT depends on a minimum glycemia to correct and a compensation factor.

12. Method for communicating treatment information to a user according to any of the claims 8 to 11, wherein the BT decreases depending on the amount of time elapsed since a previous user notification has been issued to a user or a previous bolus has been administered to the user.

13. Method for communicating treatment information to a user according to claim 12, wherein the BT has a minimum value after a certain amount of time elapsed since a previous user notification has been issued to a user or a previous bolus has been administered to the user.

14. Method for communicating treatment information to a user according to any of the claims 8 to 13, wherein a disturbance bypass is an emergency bypass, said emergency bypass being triggered wherein a risk for the user is identified from the data.

15. A computer program product comprising instructions which, when the program is executed by a computer, cause the computer to carry out the steps of the method of any of the claims 8 to 14.

[Fig. 1]

[Fig. 2]

[Fig. 3]

# EP 4 243 031 A1

Europäisches
Patentamt

European
Patent Office

Office européen
des brevets

## EUROPEAN SEARCH REPORT

Application Number

EP 23 16 1235

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/067767 A1 (BETA BIONICS INC [US]) 8 April 2021 (2021-04-08) * abstract; figures 2A, 6, 14 * * paragraph [0004] - paragraph [0009] * * paragraph [0073] - paragraphs [0081], [0089] * * paragraph [0119] - paragraph [0126] * * paragraph [0151] - paragraph [0171] * * paragraph [0199] - paragraph [0201] * * paragraph [0214] - paragraphs [0226], [0308] * * paragraphs [0413], [0487] * | 1-15 | INV. G16H20/17 A61B5/145 A61B5/00 A61M5/172 G16H40/67 G16H50/20 G16H40/63 |
| A | US 2021/050085 A1 (HAYTER GARY A [US] ET AL) 18 February 2021 (2021-02-18) * the whole document * | 1-15 | |
| A | CA 3 151 777 A1 (BETA BIONICS INC [US]) 8 April 2021 (2021-04-08) * the whole document * | 1-15 | |
| A | CN 111 655 128 A (DEXCOM INC) 11 September 2020 (2020-09-11) * the whole document * | 1-15 | TECHNICAL FIELDS SEARCHED (IPC) G16H A61B A61M |
| A | US 2019/184094 A1 (SJOLUND JOHN [US] ET AL) 20 June 2019 (2019-06-20) * the whole document * | 1-15 | |

The present search report has been drawn up for all claims

| Place of search | Date of completion of the search | Examiner |
|---|---|---|
| Munich | 31 July 2023 | Kutzarova, Iskrena |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

12

## ANNEX TO THE EUROPEAN SEARCH REPORT
## ON EUROPEAN PATENT APPLICATION NO.

EP 23 16 1235

This annex lists the patent family members relating to the patent documents cited in  the above-mentioned European search report.
The members are as contained in the European Patent Office EDP file on
The European Patent Office is in no way liable for these particulars which are merely given for the purpose of information.

31-07-2023

| Patent document cited in search report | Publication date | Patent family member(s) | Publication date |
|---|---|---|---|
| WO 2021067767 A1 | 08-04-2021 | AU 2020356952 A1 | 14-04-2022 |
| | | AU 2020358856 A1 | 14-04-2022 |
| | | CA 3151777 A1 | 08-04-2021 |
| | | CA 3151782 A1 | 08-04-2021 |
| | | CN 114868200 A | 05-08-2022 |
| | | CN 114902344 A | 12-08-2022 |
| | | DE 112020004762 T5 | 11-08-2022 |
| | | DE 112020004780 T5 | 01-09-2022 |
| | | EP 4042436 A1 | 17-08-2022 |
| | | EP 4042441 A1 | 17-08-2022 |
| | | IL 291671 A | 01-05-2022 |
| | | IL 291746 A | 01-05-2022 |
| | | JP 2022551265 A | 08-12-2022 |
| | | JP 2023503792 A | 01-02-2023 |
| | | WO 2021067767 A1 | 08-04-2021 |
| | | WO 2021067856 A1 | 08-04-2021 |
| US 2021050085 A1 | 18-02-2021 | AU 2020324387 A1 | 10-02-2022 |
| | | CA 3147267 A1 | 11-02-2021 |
| | | CN 114206207 A | 18-03-2022 |
| | | EP 4007523 A1 | 08-06-2022 |
| | | JP 2022543239 A | 11-10-2022 |
| | | US 2021050085 A1 | 18-02-2021 |
| | | WO 2021026004 A1 | 11-02-2021 |
| CA 3151777 A1 | 08-04-2021 | AU 2020356952 A1 | 14-04-2022 |
| | | AU 2020358856 A1 | 14-04-2022 |
| | | CA 3151777 A1 | 08-04-2021 |
| | | CA 3151782 A1 | 08-04-2021 |
| | | CN 114868200 A | 05-08-2022 |
| | | CN 114902344 A | 12-08-2022 |
| | | DE 112020004762 T5 | 11-08-2022 |
| | | DE 112020004780 T5 | 01-09-2022 |
| | | EP 4042436 A1 | 17-08-2022 |
| | | EP 4042441 A1 | 17-08-2022 |
| | | IL 291671 A | 01-05-2022 |
| | | IL 291746 A | 01-05-2022 |
| | | JP 2022551265 A | 08-12-2022 |
| | | JP 2023503792 A | 01-02-2023 |
| | | WO 2021067767 A1 | 08-04-2021 |
| | | WO 2021067856 A1 | 08-04-2021 |
| CN 111655128 A | 11-09-2020 | AU 2019217879 A1 | 23-07-2020 |
| | | CA 3089642 A1 | 15-08-2019 |
| | | CN 111655128 A | 11-09-2020 |
| | | EP 3749183 A1 | 16-12-2020 |

EPO FORM P0459

For more details about this annex : see Official Journal of the European Patent Office, No. 12/82

page 1 of 2

## ANNEX TO THE EUROPEAN SEARCH REPORT
### ON EUROPEAN PATENT APPLICATION NO.

**EP 23 16 1235**

This annex lists the patent family members relating to the patent documents cited in the above-mentioned European search report.
The members are as contained in the European Patent Office EDP file on
The European Patent Office is in no way liable for these particulars which are merely given for the purpose of information.

**31-07-2023**

| Patent document cited in search report | Publication date | Patent family member(s) | | Publication date |
|---|---|---|---|---|
| | | JP | 2021513136 A | 20-05-2021 |
| | | US | 2019246914 A1 | 15-08-2019 |
| | | US | 2019246973 A1 | 15-08-2019 |
| | | US | 2019251456 A1 | 15-08-2019 |
| | | US | 2019252079 A1 | 15-08-2019 |
| | | WO | 2019157102 A1 | 15-08-2019 |
| US 2019184094 A1 | 20-06-2019 | NONE | | |

For more details about this annex : see Official Journal of the European Patent Office, No. 12/82

EPO FORM P0459

**page 2 of 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7713229 B2

- US 9439602 B2